# EUROPEAN PATENT APPLICATION

(11) **EP 1 516 868 A1**
(43) Date of publication of application: **23.03.2005**
(21) Application number: 03730521.6
(22) Date of filing: 19.05.2003
(51) Int. Cl.: C07C 211/04, C07C 209/16, C07C 209/02, C07B 61/00

(54) **PROCESS FOR THE PREPARATION OF DIMETHYLAMINE**

(30) Priority: 27.06.2002 JP 2002188343
(71) Applicant: Mitsubishi Rayon Co., Ltd., Tokyo 108-8506 (JP)
(72) Inventor: HIROHATA, Jitsuo c/o Chemicals Development Lab., Yokohama-shi, Kangawa 230-0053 (JP); HOSHINO, Manabu c/o Chemicals Development Lab., Yokohama-shi, Kanagawa 230-0053 (JP); KANEKO, Isao c/o Chemicals Development Lab., Yokohama-shi, Kanagawa 2302-0053 (JP)
(74) Representative: Jones, Helen Marjorie Meredith
(86) International application number: PCT/JP2003/006216
(87) International publication number: WO 2004/002937

(57) **Abstract**

The present invention provides a new zeolite catalyst for the preparation of dimethyl amine (DMA) by the reaction of methanol and/or a methylamine mixture with ammonia, which solves the problems involved in a conventional catalyst resulting from sharply declined DMA selectivity as methanol conversion reaches 95% or higher, to exhibit a higher DMA selectivity, lower trimethylamine selectivity and higher methanol-depleting reaction activity.

The process of the present invention prepares dimethylamine by a vapor-phase reaction of methanol and/or a methylamine mixture with ammonia in the presence of an improved zeolite catalyst prepared by treating a zeolite with a solution dissolving an organophosphorus compound.

## Description

### FIELD OF THE INVENTION

The present invention relates to a process for the preparation of dimethylamine by a vapor-phase catalytic reaction of methanol and/or a methylamine mixture with ammonia, more particularly a process for the preparation of dimethylamine in the presence of an improved zeolite catalyst. Diethylamine is an important chemical intermediate as a starting material for various solvents, medicines, rubber chemicals, surfactants and so on.

Dimethylamine is generally prepared by reacting methanol with ammonia at high temperature (around 400°C) in the presence of a solid acid catalyst having a dehydration and amination functions, e.g., alumina or silica-alumina. This reaction prepares monomethylamine (hereinafter referred to as MMA) and trimethylamine (hereinafter referred to as TMA) as by-products, in addition to dimethylamine (hereinafter referred to as DMA). These by-product methylamines, which are demanded much less than DMA, are separated from the reaction effluent stream and are recycled back to the reaction system for reuse.

### BACKGROUND OF THE INVENTION

Distillation is carried out for separating dimethylamine from reaction products of methylamine. However, this needs a very complicated and large-size distillation operation, because TMA forms a very complex azeotrope with ammonia, MMA and DMA. As a result, the DMA recovery process consumes a large quantity of energy cost. This recovery processes are described in detail in, e.g., "Revised Production Flow Sheets," issued on April 25, 1978 by Kagaku Kogyo Ltd., Japan.

In order to reduce DMA preparation cost and system size, it is essential to accelerate preparation of DMA and to suppress a formation of by-product methylamines, in particular TMA. However, selectivities of these three types of methylamines in the presence of a usual amorphous solid acid catalyst, e.g., alumina or silica-alumina, are determined by thermodynamics, and TMA is prepared at a much higher production yield than DMA under the normal reaction conditions.

Under conditions of 400°C as reaction temperature and 1/1 (by weight) as ammonia/methanol ratio at a reactor inlet, for example, the thermodynamically determined equilibrium MMA/DMA/TMA ratio is 0.284/0.280/0.436 by weight. It is therefore invariably necessary to separate large quantities of MMA and TMA prepared, and to recycle them back to the reaction system together with a large quantity of ammonia, which is present in excess of the stoichiometric requirement to favor the equilibrium DMA-preparation reaction.

Recently, various types of zeolite catalysts have been proposed to solve these problems. For example, these are disclosed by JP-A-56-69846 for zeolite A, JP-A-54-148708 and JP-A-58-69846 for FU-1, USP 4,082,805 for ZSM-5, JP-A-56-113746 for ferrierite and erionite, JP-A-61-178951 and JP-A-63-8358 for rho, ZK-5 and shabasite, and JP-A-56-46846, JP-A-59-210050 and JP-A-58-049340 for mordenite.

All of the processes which use these zeolite catalysts give a DMA selectivity exceeding a thermodynamic equilibrium level. However, their DMA selectivity and suppression of TMA formation are not always sufficient. There is another problem of needing a fairly large quantity of unreacted methanol kept in the reaction system for maintaining a high DMA selectivity, because DMA selectivity normally declines sharply as methanol conversion exceeds 95 to 96%. For example, JP-A-59-210050 discloses a process for selectively preparation of DMA, wherein the reaction proceeds in the presence of Na-mordenite while keeping the methanol conversion at 80 to 96%. The zeolite catalyst used in this process favorably compares with those previously proposed in DMA selectivity and methanol-depleting reaction activity. It produces the following favorable results under an N/C ratio of 1 to 2.5 and methanol conversion of 80% or more, which are normally considered to be the preferable levels; DMA: 53.0% and TMA: 7.7% (methanol conversion: 86.1% and SV: 2010), and DMA: 53.9% and TMA: 12.9% (methanol conversion: 94.1% and SV: 2020), by weight %.

Moreover, activity (methanol-depleting reaction rate) is frequently incompatible with selectivity. In other words, a high selectivity is realized at the sacrifice of activity to some extent, and so is vice versa. For example, JP-A-59-210050 cited above discloses in EXAMPLE 1 that reaction activity declines from SV2010 to SV1010 at a methanol conversion of around 90% as DMA preparation rate is increased from 39.5% to 49.3% by weight by increasing alkali cation quantity. Selective preparation of DMA by the use of a zeolite catalyst is described in detail in "Catalyst, Vol. 29, No. 4, P. 322, Japan".

The following methylamine preparation processes are known for improving DMA selectivity in the presence of a modified zeolite catalyst. JP-A-61-254256, in particular, discloses a process which uses a zeolite catalyst of chabasite, erionite, zeolite rho or zeolite ZK-5 modified with a compound containing at least one element selected from the group consisting of silicon, aluminum, phosphorus and boron, deposited on the zeolite. This process, however, involves problems of increased consumption of the modifier, because it is used without being diluted, and insufficient reaction results, e.g., low activity, low rate of methylamine preparation from methanol, and massive formation of by-products, e.g., dimethyl ether. JP-A-11-35527 discloses a process which uses a crystalline silicoaluminophosphate, silylation-treated in a liquid phase. The following processes are known for mordenite. JP-A-59-227841 discloses a process which uses a steam-treated mordenite catalyst, JP-A-6-179640 discloses a process which uses a mordenite catalyst silylation-treated in a liquid phase, JP-A-3-262540 discloses a process which uses a mordenite catalyst treated with SiCl₄ in a vapor phase, JP-A-8-225498 discloses a process which uses a mordenite catalyst treated with a solution containing a chelating agent, and JP-A-2000-302735 discloses a process which uses a mordenite catalyst ion-exchanged with aluminum.

For DMA preparation by the use of Na-mordenite, JP-A-56-46846 discloses mordenite with a controlled Na content for selective preparation of DMA from MMA, and JP-A-59-210050 also discloses mordenite with a controlled Na content for selective preparation of DMA. For DMA preparation by the use of high-silica mordenite, JP-A-6-9510 discloses Mg-containing high-silica mordenite.

### DISCLOSURE OF THE INVENTION

As discussed above, various zeolite catalysts have been proposed for the preparation of methylamine. However, they are still insufficient, and there are demands for further improved or developed zeolite catalysts which can give DMA at a higher yield while suppressing formation of TMA in the above-described reaction. It is an object of the present invention to provide a process for the preparation of dimethylamine by the use of a new zeolite catalyst by the reaction of methanol and/or a methylamine mixture with ammonia, for which the conventional zeolite catalyst involving the problems resulting from sharply declined DMA selectivity, when methanol conversion reaches 95% or higher, is improved to exhibit a higher DMA selectivity, lower TMA selectivity and higher methanol-depleting reaction activity.

The inventors of the present invention have found, after having extensively studied to fulfill the above object, that a modified zeolite can exhibit a very high DMA selectivity, low TMA selectivity and high methanol-depleting reaction activity as a catalyst for the preparation of dimethylamine by the reaction of methanol and/or a methylamine mixture with ammonia, when the modified zeolite is prepared by treating a zeolite with a solution dissolving an organophosphorus compound for modification, achieving the present invention.

The present invention relates to a process for the preparation of dimethylamine by a vapor-phase reaction of methanol and/or a methylamine mixture with ammonia by the use of a modified zeolite catalyst, which is prepared by treating a zeolite with a solution dissolving an organophosphorus compound for modification.

### BEST MODE FOR CARRYING OUT THE INVENTION

The present invention is described in detail below. It is important for the modified zeolite catalyst for the process of the present invention to have been treated with a solution dissolving an organophosphorus compound.

A zeolite used in the present invention is preferably selected from those exhibiting a molecular shape-selectivity in the reaction between ammonia and methanol for forming methylamine. These zeolites include mordenite, clinoptilolite, levynite, zeolite A, FU-1, ZSM-5, ZSM-11, ZSM-21 and montmorillonite, of which mordenite is particularly preferable. Of mordenites, preferable ones include mordenites containing sodium at 2 g or less, more preferably 0.01 to 2 g, per 100 g and hydrogen type mordenite. Still more preferable ones include mordenites having a Si/Al atomic molar ratio adjusted at 5.5 to 9, still more preferably 5.5 to 7.

The organophosphorus compounds used for treating the zeolite include phosphates, e.g., trimethyl phosphate, triethyl phosphate, tributyl phosphate and triphenyl phosphate; acid phosphates, e.g., methyl acid phosphate and ethyl acid phosphate; phosphites, e.g., trimethyl phosphite, diethyl phosphite and triethyl phosphite; phosphonic acids, e.g., phenyl phosphonic acid; phosphines, e.g., tributyl phosphine and triphenyl phosphine; and phosphine oxides, e.g., triphenyl phosphine oxide. Of these, phosphates, phosphites, phosphines and phosphine oxides are more preferable, and trimethyl phosphate, triethyl phosphate, tributyl phosphate, triethyl phosphite, tributyl phosphine, triphenyl phosphine and triphenyl phosphine oxide are still more preferable. The particularly preferable ones are trimethyl phosphate, triethyl phosphate, tributyl phosphate and triethyl phosphite.

The zeolite is treated with a solution dissolving an organophosphorus compound. The solvents useful for dissolving the organophosphorus compound include aliphatic and alicyclic hydrocarbons, e.g., hexane, heptane, octane and cyclohexane; aromatic hydrocarbons, e.g., benzene, toluene and xylene; ethers, e.g., diethyl ether and diisopropyl ether; ketones, e.g., acetone and methylethylketone; and esters, e.g., ethyl acetate and butyl acetate. Of these, aliphatic, alicyclic and aromatic hydrocarbons are more preferable, and toluene is particularly preferable. For modification, the zeolite is normally immersed in, or stirred together with, a solution dissolving an organophosphorus compound, or the solution is passed over the zeolite. The organophosphorus compound is used preferably at 0.001 to 20 mols per 100 g of the zeolite, more preferably 0.01 to 10 mols. Concentration of the organophosphorus compound is preferably 0.001 to 20 mols/liter, more preferably 0.01 to 10 mols/liter. Modification treatment temperature is preferably room temperature to 110°C, more preferably 40 to 110°C, still more preferably 60 to 110°C. Treatment period of time is preferably 1 to 500 hours, more preferably 2 to 100 hours, still more preferably 1 to 10 hours.

The zeolite catalyst modified with an organophosphorus compound contains phosphorus preferably at 0.02 to 5% by weight, more preferably 0.05 to 1% by weight.

The zeolite catalyst for the present invention, modified with an organophosphorus compound, exhibits a higher DMA selectivity than that treated with a phosphorus compound other than the above, e.g., phosphorous trichloride. The treatment with an organophosphorus compound brings a particularly favorable effect, when applied to mordenite having a Si/Al atomic ratio of 5.5 to 9.

The reaction conditions for the preparation of dimethylamine by the present invention are temperature of preferably 200 to 350°C, more preferably 230 to 350°C, still more preferably 250 to 330°C; pressure of preferably normal pressure to 50 Kg/cm²G, more preferably 5 to 50 Kg/cm²G, still more preferably 5 to 30 Kg/cm²G; N/C ratio (nitrogen/carbon atomic ratio in the reaction system) of preferably 1 to 2.5, more preferably 1.5 to 2.3; space velocity of preferably 600 to 3200/hour, more preferably 600 to 2000/hour; and methanol conversion of preferably 80 to 98%, more preferably 85 to 97%.

The present invention is described in more detail by Examples and Comparative Examples, which by no means limit the present invention.

### Reaction testing method

A 50/50 by weight mixture of ammonia and methanol was passed over the catalyst packed in a stainless steel reactor tube (800 mm in length and 0.5 inch in diameter) at a rate of 1.05 g/minute under conditions of 322°C as reaction temperature and 17.5 Kg/cm²G as pressure.

### Example 1

Powdery Na type mordenite was treated with a 20 times larger quantity of 3N boiling solution of ammonium nitrate with reflux for 6 hours, and mordenite was separated by filtration. This procedure was repeated 4 times. The separated mordenite was washed with water, dried at 130°C for 6 hours, and calcined at 450°C for 3 hours to prepare the H type (hydrogen type) mordenite. Then, 100 g of the H type mordenite was treated in 1L (liter) of 1N boiling solution of sodium nitrate at 40°C for 20 hours with reflux, to prepare a mordenite containing sodium at 0.4% by weight. The H type mordenite had a Si/Al atomic molar ratio of 6.0. This mordenite was formed into cylindrical pellets, 3 mm in diameter. Then, 50 g of the H type mordenite was immersed in 500 g of a toluene solution dissolving 50g of triethyl phosphate, kept at 100°C, for 4 hours. The treated mordenite was washed with toluene, dried at 80°C for 4 hours, and calcined at 570°C for 4 hours. The resulting modified mordenite catalyst contained phosphorus at 0.27% by weight. The resulting modified mordenite catalyst was used to prepare dimethylamine by the reaction between ammonia and methanol. Composition of the resulting methylamine mixture is given in Table 1. No by-products, e.g., dimethylether and the like, were observed.

### Example 2

A modified mordenite catalyst was prepared and was used for the reaction in the same manner as in Example 1, except that 50 g of the H type mordenite prepared in Example 1 was treated with 500 g of a toluene solution dissolving 150 g of triethyl phosphate. The resulting modified mordenite catalyst contained phosphorus at 0.33% by weight. Composition of the resulting methylamine mixture is given in Table 1. No by-products, e.g., dimethylether and the like, were observed.

### Example 3

A modified mordenite catalyst was prepared and was used for the reaction in the same manner as in Example 1, except that 50 g of the H type mordenite prepared in Example 1 was treated with 500 g of a toluene solution dissolving 250 g of triethyl phosphate. The resulting modified mordenite catalyst contained phosphorus at 0.36% by weight. Composition of the resulting methylamine mixture is given in Table 1. No by-products, e.g., dimethylether and the like, were observed.

### Example 4

A modified mordenite catalyst was prepared and was used for the reaction in the same manner as in Example 1, except that 50 g of the H type mordenite prepared in Example 1 was treated with 500 g of a toluene solution dissolving 10 g of triethyl phosphate. The resulting modified mordenite catalyst contained phosphorus at 0.20% by weight. Composition of the resulting methylamine mixture is given in Table 1. No by-products, e.g., dimethylether and the like, were observed.

### Example 5

A modified mordenite catalyst was prepared and was used for the reaction in the same manner as in Example 1, except that 50 g of the H type mordenite prepared in Example 1 was treated with 500 g of a toluene solution dissolving 25 g of triethyl phosphate. The resulting modified mordenite catalyst contained phosphorus at 0.22% by weight. Composition of the resulting methylamine mixture is given in Table 1. No by-products, e.g., dimethylether and the like, were observed.

### Example 6

A modified mordenite catalyst was prepared and was used for the reaction in the same manner as in Example 1, except that 50 g of the H type mordenite prepared in Example 1 was treated with 500 g of a toluene solution dissolving 50 g of trimethyl phosphate. The resulting modified mordenite catalyst contained phosphorus at 0.33% by weight. Composition of the resulting methylamine mixture is given in Table 1. No by-products, e.g., dimethylether and the like, were observed.

### Example 7

A modified mordenite catalyst was prepared and was used for the reaction in the same manner as in Example 1, except that 50 g of the H type mordenite prepared in Example 1 was treated with 500 g of a toluene solution dissolving 50 g of tributyl phosphate. The resulting modified mordenite catalyst contained phosphorus at 0.29% by weight. Composition of the resulting methylamine mixture is given in Table 1. No by-products, e.g., dimethylether and the like, were observed.

### Example 8

A modified mordenite catalyst was prepared and was used for the reaction in the same manner as in Example 1, except that 50 g of the H type mordenite prepared in Example 1 was treated with 500 g of a toluene solution dissolving 50 g of triethyl phosphite. The resulting modified mordenite catalyst contained phosphorus at 0.37% by weight. Composition of the resulting methylamine mixture is given in Table 1. No by-products, e.g., dimethylether and the like, were observed.

### Example 9

A modified mordenite catalyst was prepared and was used for the reaction in the same manner as in Example 1, except that 50 g of the H type mordenite prepared in Example 1 was treated with 500 g of a toluene solution dissolving 50 g of tributyl phosphine. The resulting modified mordenite catalyst contained phosphorus at 0.22% by weight. Composition of the resulting methylamine mixture is given in Table 1. No by-products, e.g., dimethylether and the like, were observed.

### Example 10

A modified mordenite catalyst was prepared and was used for the reaction in the same manner as in Example 1, except that 50 g of the H type mordenite prepared in Example 1 was treated with 500 g of a toluene solution dissolving 50 g of triphenyl phosphine. The resulting modified mordenite catalyst contained phosphorus at 0.10% by weight. Composition of the resulting methylamine mixture is given in Table 1. No by-products, e.g., dimethylether and the like, were observed.

### Example 11

A modified mordenite catalyst was prepared and was used for the reaction in the same manner as in Example 1, except that 50 g of the H type mordenite prepared in Example 1 was treated with 500 g of a toluene solution dissolving 50 g of triphenyl phosphine oxide. The resulting modified mordenite catalyst contained phosphorus at 0.10% by weight. Composition of the resulting methylamine mixture is given in Table 1. No by-products, e.g., dimethylether and the like, were observed.

**Table 1**

| Examples | Organophosphorus compound | Space velocity (1/hour) | Methanol conversion (%) | Content of each methylamine in the methylamine product (%) | |
|---|---|---|---|---|---|
| | | | | DMA | TMA |
| 1 | Triethyl phosphate | 3200 | 84.5 | 60.3 | 5.6 |
| | | 1600 | 97.0 | 62.7 | 8.6 |
| 2 | Triethyl phosphate | 3200 | 81.0 | 60.2 | 5.1 |
| | | 1600 | 96.2 | 62.9 | 8.5 |
| 3 | Triethyl phosphate | 3200 | 85.4 | 61.3 | 5.8 |
| | | 1600 | 97.2 | 63.1 | 8.4 |
| 4 | Triethyl phosphate | 3200 | 89.2 | 60.2 | 6.2 |
| | | 1600 | 98.0 | 60.6 | 10.2 |
| 5 | Triethyl phosphate | 3200 | 86.9 | 62.0 | 6.1 |
| | | 1600 | 97.2 | 60.2 | 9.0 |
| 6 | Trimethyl phosphate | 3200 | 84.6 | 60.5 | 4.9 |
| | | 1600 | 96.5 | 63.0 | 7.6 |
| 7 | Tributyl phosphate | 3200 | 82.3 | 60.0 | 4.5 |
| | | 1600 | 96.9 | 62.1 | 8.3 |
| 8 | Trietyl phosphite | 3200 | 81.8 | 59.3 | 5.3 |
| | | 1600 | 96.1 | 61.9 | 8.4 |
| 9 | Tributyl phosphine | 3200 | 82.1 | 60.3 | 5.7 |
| | | 1600 | 96.7 | 62.9 | 8.8 |
| 10 | Triphenyl phosphine | 3200 | 85.7 | 58.6 | 7.8 |
| | | 1600 | 97.6 | 58.2 | 12.1 |
| 11 | Triphenyl | 3200 | 83.7 | 58.6 | 8.4 |
| | phosphine oxide | 1600 | 96.9 | 58.1 | 12.4 |

### Comparative Example 1

Mordenite containing sodium at 0.4% was prepared in the same manner as in Example 1, and was formed into cylindrical pellets, 3 mm in diameter. The resulting H type mordenite catalyst was used to prepare dimethylamine by the reaction between ammonia and methanol, without having been treated with an organophosphorus compound. The resulting catalyst contained phosphorus at 0.00% by weight. Composition of the resulting methylamine mixture is given in Table 2.

### Comparative Example 2

The H type mordenite prepared in Example 1 (25 g) was immersed in 500 g of a toluene solution dissolving 25 g of phosphorous trichloride, kept at 70°C, for 4 hours. The treated mordenite was washed with water, was dried at 130°C for 4 hours and was calcined at 570°C for 4 hours. The resulting modified mordenite catalyst contained phosphorus at 0.17% by weight and was used to prepare dimethylamine by the reaction between ammonia and methanol. Composition of the resulting methylamine mixture is given in Table 2.

### Comparative Example 3

The H type mordenite prepared in Example 1 (50 g) was immersed in 500 mL of a 1N aqueous solution of phosphoric acid, kept at 80°C, for 8 hours. The treated mordenite was washed with water, was dried at 80°C for 4 hours, and was calcined at 570°C for 4 hours. The resulting modified mordenite catalyst contained phosphorus at 0.18% by weight and was used to prepare dimethylamine by the reaction between ammonia and methanol. Composition of the resulting methylamine mixture is given in Table 2.

**Table 2**

| Comparative Examples | Modifier | Space velocity (1/hour) | Methanol conversion (%) | Content of each methylamine in the methylamine product (%) | |
|---|---|---|---|---|---|
| | | | | DMA | TMA |
| 1 | Not used | 1400 | 96.8 | 34.1 | 35.2 |
| 2 | Phosphorous trichloride | 1600 | 97.5 | 53.4 | 18.8 |
| 3 | Phosphoric acid | 1600 | 97.5 | 57.7 | 15.5 |

### INDUSTRIAL APPLICABILITY

The modified zeolite catalyst for the present invention, treated with a solution containing an organophosphorus compound, exhibits an improved DMA selectivity and suppressed TMA selectivity as a dimethylamine preparation catalyst while keeping a high dimethylamine selectivity even at a high methanol-depleting reaction activity. As a result, it can exhibit a higher dimethylamine selectivity even at a high methanol conversion of 98% than a conventional zeolite catalyst for the preparation of dimethylamine by the reaction of methanol and/or a methylamine mixture with ammonia and, moreover, can exhibit a greatly improved catalyst life.

## Claims

1. A process for preparing dimethylamine by a vapor-phase reaction of methanol and/or a methylamine mixture with ammonia in the presence of a zeolite catalyst, comprising the step of:
carrying out the reaction in the presense of a modified zeolite catalyst which is prepared by treating a zeolite with a solution dissolving an organophosphorus compound.

2. The process according to Claim 1, wherein the zeolite is mordenite.

3. The process according to Claim 1 or 2, wherein the organophosphorus compound is at least one type selected from the group consisting of phosphates, phosphites, phosphines, phosphine oxides.

4. The process according to any one of Claims 1 to 3, wherein the zeolite is mordenite containing 2 g or less of sodium per 100 g.

5. The process according to any one of Claims 1 to 4, wherein the zeolite is mordenite having a Si/Al atomic molar ratio of 5.5 to 9.

6. The process according to any one of Claims 1 to 5, wherein the vapor-phase reaction is carried out under conditions of a temperature of 200 to 350°C, a pressure of normal pressure to 50 Kg/cm²G, an N/C ratio (nitrogen/carbon atomic molar ratio in the reaction system) of 1.2 to 2.5, and a methanol conversion of 80 to 98%.
